(19) Europäisches Patentamt
European Patent Office
Office européen des brevets

(11) EP 1 435 903 B1

(12) EUROPEAN PATENT SPECIFICATION

(45) Date of publication and mention of the grant of the patent: **01.06.2005 Bulletin 2005/22**

(21) Application number: **02768794.6**

(22) Date of filing: **04.09.2002**

(51) Int Cl.[7]: **A61K 7/50**, C11D 3/32, C11D 1/66, C11D 1/52

(86) International application number: **PCT/US2002/028153**

(87) International publication number: **WO 2003/022240 (20.03.2003 Gazette 2003/12)**

(54) **CLEANSING COMPOSITION**

REINIGUNGSZUSAMMENSETZUNG

COMPOSITION DE NETTOYAGE

(84) Designated Contracting States:
**AT BE BG CH CY CZ DE DK EE ES FI FR GB GR IE IT LI LU MC NL PT SE SK TR**
Designated Extension States:
**RO**

(30) Priority: **06.09.2001 US 947828**

(43) Date of publication of application: **14.07.2004 Bulletin 2004/29**

(73) Proprietor: **Colgate-Palmolive Company**
**New York, N.Y. 10022 (US)**

(72) Inventor: **HARMALKER, Subhash**
**Somerset, NJ 08873 (US)**

(74) Representative:
**Winckels, Johannes Hubertus F. et al**
**Vereenigde**
**Nieuwe Parklaan 97**
**2587 BN Den Haag (NL)**

(56) References cited:
**WO-A-00/42984**
**WO-A-99/57238**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 435 903 B1

## Description

### BACKGROUND OF THE INVENTION

Fluid compositions capable of cleansing surfaces, including the skin, and also having antibacterial activity have been commercially available for many years. Generally, the criteria for a successful antibacterial in such a system is solubilization into the system, maintain stability of the system, precipitate out when water is added to the system and deposit on surface being treated while maintaining antibacterial efficacy.

Certain antibacterial agents satisfy these criteria relatively easily while others have varying degrees of difficulty meeting these criteria. One of the hardest to meet all these criteria in a fluid system are antibacterial agents that are members of the carbanilide family, more specifically triclocarban, hereafter referred to as TCC. It is quite difficult to solubilize TCC and then remove it from the system while having efficacy on the surface, as well as maintaining stability of the system during shelf life.

WO-A- 0 042 984 discloses aqueous liquid cleansing compositions comprising trichlorocarbanilide and an alkanolamide surfactant, such as cocamido MEA.

It has now been discovered that use of a specific solubilizer preferably together with a second solubilizer brings about a fluid cleansing system with a carbanilide antibacterial which is a stable system having a solubilized carbanilide, and which has antibacterial efficacy when precipitated onto a surface through the addition of water.

### SUMMARY OF THE INVENTION

In accordance with the invention, there is an aqueous fluid composition, which comprises:

a. a cleansing effective amount of a surfactant or mixture thereof,
b. an antibacterial effective amount of a carbanilide compound or mixture thereof,
c. at least about 0.25 wt. % of composition of a compound or mixture thereof of the formula

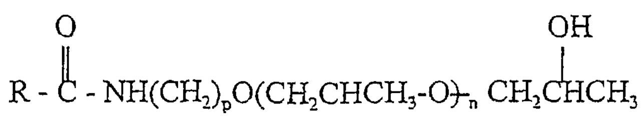

wherein R is alkyl or alkenyl of about 8 to about 20 carbon atoms, n is an integer of 1 to about 8, and p is 2 or 3.

A further aspect of the invention is an aqueous, fluid composition, which comprises:

a. at least about 5 wt. % of a surfactant or mixture thereof,
b. at least about .03 wt. % of an antibacterial carbanilide or mixture thereof,
c. at least about 0.25 wt. % of composition of a compound or mixture thereof of the formula

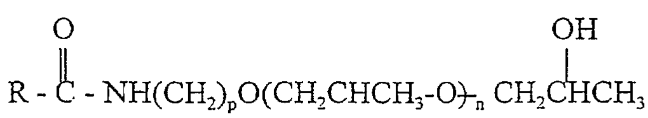

wherein R is alkyl or alkenyl of about 8 to about 20 carbon atoms, n is an integer of 1 to about 8, and p is 2 or 3.

Another aspect of the invention is applying a composition as identified above after or concomitantly with dilution with water to a surface, particularly skin, and obtaining a combination of surface cleansing and antibacterial efficacy.

A still further aspect of the invention is a fluid system of a fragrance, a halogenated carbanilide, a compound or mixture thereof of the formula

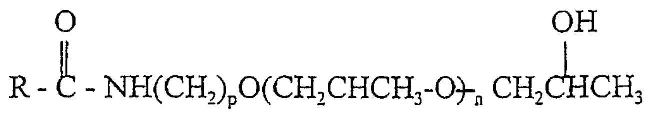

wherein R is alkyl or alkenyl of about 8 to about 20 carbon atoms, n is an integer of 1 to about 8, and p is 2 or 3, and, optionally, an alcohol or mixture thereof of the formula,

$$R'(CH_2CH_2O)_qH$$

wherein R' is alkyl or alkenyl of 10 to 15 carbon atoms and q is 7, 8 or 9.

## DETAILED DESCRIPTION OF THE INVENTION

The aqueous fluid system is generally a liquid or a gel. Such system should remain the same visually with respect to clarity, separation, precipitate and the like with the surfactant, the antibacterial agents and stabilizing solubilizing material therein over a specified period of time to be called stable in comparison to the composition without the stabilizing, solubilizing material therein.

The surface to be cleansed can be any hard surface such as a sink, toilet, countertop and dish found in the home, or a person's skin. Personal care products for skin cleansing are preferred.

Any surfactant or mixture of surfactants at an effective amount for cleansing can be employed.

The surfactant can be anionic, nonionic, amphoteric, or cationic, preferably anionic. Soap, a long chain alkyl or alkenyl, branched or normal carboxylic acid salt such as sodium, potassium, ammonium or substituted ammonium salt can be present in the composition as an example of an anionic surfactant. Exemplary of long chain alkyl or alkenyl are from about 8 to about 22 carbon atoms in length, specifically about 10 to about 20 carbon atoms in length, more specifically alkyl and most specifically normal, or normal with little branching. Small quantities of olefinic bond(s) may be present in the predominantly alkyl sections, particularly if the source of the "alkyl" group is obtained from a natural product such as tallow, coconut oil and the like. Because of its potential harshness soap is not a preferred surfactant and can be omitted from the composition.

Other surfactants can be present in the composition as well. Examples of such surfactants are the anionic, amphoteric, nonionic and cationic surfactants. Examples of anionic surfactants include but are not limited to soaps, alkyl sulfates, anionic acyl sarcosinates, methyl acyl taurates, N-acyl glutamates, acyl isethionates, alkyl sulfosuccinates, alkyl phosphate esters, ethoxylated alkyl phosphate esters, trideceth sulfates, protein condensates, mixtures of ethoxylated alkyl sulfates and the like.

Alkyl chains for these surfactants are $C_8$-$C_{22}$, preferably $C_{10}$-$C_{18}$, more preferably $C_{12}$-$C_{14}$.

Anionic non-soap surfactants can be exemplified by the alkali metal salts of organic sulfate having in their molecular structure an alkyl radical containing from about 8 to about 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$-$C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art,

Zwitterionic surfactants can be exemplified by those which can be broadly described as derivatives of aliphatic quaternary ammonium, phosphonium, and sulfonium compounds, in which the aliphatic radicals can be straight chain or branched and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

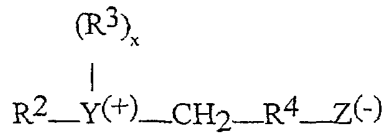

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from the group consisting of nitrogen, phosphorus, and sulfur atoms; R3 is an alkyl or monohydroxyalkyl group containing 1 to about 3 carbon atoms; X is 1 when Y is a sulfur atom and 2 when Y is a nitrogen or phosphorus atom, $R^4$ is an alkylene or hydroxyalkylene of from 0 to about 4 carbon atoms and Z is a radical selected from the group consisting of carboxylate, sulfonate, sulfate, phosphonate, and phosphate groups.

Examples include: 4-[N,N-di(2-hydroxyethyl)-N-octadecylammonio]-butane-1-carboxylate; 5-[S-3-hydroxypropyl-S-hexadecylsulfonio] -3 hydroxypentane-1-sulfate; 3-[P,P-P-diethyl-P 3,6,9 trioxatetradecyl- phosphonio]-2-hydroxypropane-1-phosphate; 3-[N,N-dipropyl-N-3 dodecoxy-2-hydroxypropylammonio]-propane-1-phosphonate; 3-(N,N-di- methyl-N-hexadecylammonio) propane-1-sulfonate; 3-(N,N-dimethyl-N-hexadecylammonio)-2-hydroxypropane-1-sulfonate; 4-(N,N-di(2-hydroxyethyl)-N-(2 hydroxydodecyl) ammonio]-butane-1-carboxylate; 3-[S-ethyl-S-(3-dodecoxy-2-hydroxypropyl)sulfonio]-propane-1-phosphate; 3-(P,P-dimethyl-P-dodecylphosphonio)-propane-1-phosphonate; and 5-[N,N-di(3-hydroxypropyl)-N-hexadecylammonio]-2-hydroxy-pentane-1-sulfate.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines, such as the one prepared by reacting dodecylamine with sodium isethionate according to the teaching of U.S. Patent No.2,658,072, N-higher alkyl aspartic acids, such as those produced according to the teaching of U.S. Patent No. 2,438,091, and the products sold under the trade name "Miranol" and described in U.S. Patent No. 2,528,378. Other amphoterics such as betaines are also useful in the present composition.

Examples of betaines useful herein include the high alkyl betaines such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl) carboxymethyl betaine, oleyl dimethyl gamma-carboxypropyl betaine, lauryl bis-(2-hydro-xypropyl) alpha-carboxyethyl betaine, etc. The sulfobetaines may be represented by coco dimethyl sulfopropyl betaine, stearyl dimethyl sulfopropyl betaine, amido betaines, amidosulfobetaines, and the like.

Many cationic surfactants are known to the art. By way of example, the following may be mentioned:

- stearyldimenthylbenzyl ammonium chloride;
- dodecyltrimethylammonium chloride;
- nonylbenzylethyldimethyl ammonium nitrate;
- tetradecylpyridinium bromide;
- laurylpyridinium chloride;
- cetylpyridinium chloride
- laurylpyridinium chloride;
- laurylisoquinolium bromide;
- ditallow(Hydrogenated)dimethyl ammonium chloride;
- dilauryldimethyl ammonium chloride; and stearalkonium chloride.

Additional cationic surfactants are disclosed in USP 4,303,543 see column 4, lines 58 and column 5, lines 1-42, incorporated herein by references. Also see CTFA Cosmetic Ingredient Dictionary, 4th Edition 1991, pages 509-514 for various long chain alkyl cationic surfactants; incorporated herein by references.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide

per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from about 5,000 to about 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration with ethylene oxide, e.g., a coconut alcohol ethylene oxide condensate having from 10 to 30 moles of ethylene oxide per mole of coconut alcohol, the coconut alcohol fraction having from 10 to 14 carbon atoms. Other ethylene oxide condensation products are ethoxylated fatty acid esters of polyhydric alcohols (e.g., Tween 20-polyoxyethylene (20) sorbitan monolaurate).

4. Long chain tertiary amine oxides corresponding to the following general formula:

$$R_1R_2R_3N \rightarrow 0$$

wherein $R_1$ contains an alkyl, alkenyl or monohydroxy alkyl radical of from about 8 to about 18 carbon atoms, from 0 to about 10 ethylene oxide moieties, and from 0 to 1 glyceryl moiety, and, $R_2$ and $R_3$ contain from 1 to about 3 carbon atoms and from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyl-di(2-hydroxyethyl) amine oxide, dimethyloctylamine oxide, dimethyldecylamine oxide, dimethyltetradecylamine oxide, 3,6,9 trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

5. Long chain tertiary phosphine oxides corresponding to the following general formula:

$$RR'R''P \rightarrow 0$$

wherein R contains an alkyl, alkenyl or monohydroxyalkyl radical ranging from 8 to 20 carbon atoms in chain length, from 0 to about 10 ethylene oxide moieties and from 0 to 1 glyceryl moiety and R' and R" are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecylmethylethylphosphine oxide, 3,6,9-trioxaoctadecyldimethylphosphine oxide, cetyldimethylphosphine oxide, 3-dodecoxy-2-hydroxypropyldi(2-hydroxyethyl) phosphine oxide stearyldimethylphosphine oxide, cetylethyl propylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl)phosphine oxide, tetradecylmethyl-2-hydroxypropylphosphine oxide, oleyldimethylphosphine oxide, 2-hydroxydodecyldimethylphosphine oxide.

6. Long chain dialkyl sulfoxides containing one short chain alkyl or hydroxy alkyl radical of 1 to about 3 carbon atoms (usually methyl) and one long hydrophobic chain which contain alkyl, alkenyl, hydroxy alkyl, or keto alkyl radicals containing from about 8 to about 20 carbon atoms, from 0 to about 10 ethylene oxide moieties and from 0 to 1 glyceryl moiety. Examples include: octadecyl methyl sulfoxide, 2-ketotridecyl methyl sulfoxide, 3,6,9-trioxaoctadecyl 2-hydroxyethyl sulfoxide, dodecyl methyl sulfoxide, oleyl 3-hydroxypropyl sulfoxide, tetradecyl methyl sulfoxide, 3 methoxytridecylmethyl sulfoxide, 3-hydroxytridecyl methyl sulfoxide, 3-hydroxy-4-dodecoxybutyl methyl sulfoxide.

7. Alkylated polyglycosides wherein the alkyl group is from about 8 to about 20 carbon atoms, preferably about 10 to about 18 carbon atoms and the degree of polymerization of the glycoside is from about 1 to about 3, preferably about 1.3 to about 2.0.

The antibacterial agent employed in the invention is a member of the carbanilide family. Illustrative examples of the carbanilide family include carbanilide per se; 3'-trifluoromethyl-4,4'dichlorocarbanilide,3,3',4'-trichlorocarbanilide, and the like. Most preferred is the halogenated carbanilide known as triclocarban, also known as 3,4,4'-trichlorocarbanilide.

The agent(s) which bring about the aforementioned desired characteristics of the formulation, solubilization of the antibacterial carbanilide, stability of the solubilized carbanilide containing formulation, and upon water dilution the deposition of the efficacious antibacterial upon the surface, particularly skin, is an effective amount of the antibacterial compound or mixture thereof of compound(s) of the formula:

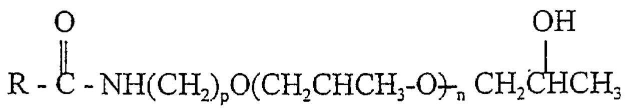

wherein R is alkyl or alkenyl of about 8 to 20 carbon atoms and mixture thereof, p is 2 or 3, and n is an integer of 1 to about 8. Examples of R groups, both branched and normal include octyl, decyl, isodecyl, 2,2,4 trimethylpentyl-4-2-ethyl decyl, dodecyl, myristyl, stearyl, palmityl, eicosyl, isostearyl, and the like including cocoyl and other naturally occurring mixtures as well as corresponding alkenyl compounds. The integer n can be 1, 2, 3, 4, 5, 6, 7 or 8, more preferred n is 1, 2, 3, or 4 and most preferred is n is 1. p is preferably 2. A preferred R is alkyl and/or alkenyl of about 12 to about 16 carbon atoms, preferably a mixture such as cocoyl. The most preferred compound is PPG-2 hydroxyethyl cocoamide, wherein R is cocoyl , p is 2, and n is 1, available from Mona Industries as Promidium CO.

It is preferred to use a cosolubilizer with the above amides. This cosolubilizer is a long chain ethoxylated primary alcohol of the formula,

$$R'(CH_2CH_2O)_qH$$

wherein R, is alkyl and/or alkenyl, preferably alkyl, of about ten to fifteen carbon atoms and q is an integer of 7, 8, or 9, preferably 7. The most preferred compound is laureth-7. The use of the cosolubilizer allows the combination of solubilizers to bring about dissolving of TCC in a formulation at room temperature. With the amide alone, heat must be applied to the formulation to bring about proper dissolving of the carbanilides. Chlorinated carbanilides, TCC in particular, upon heating can breakdown into chloroanilines, a particular undesired breakdown product because of its potential carcinogenicity. Therefore, the ability to process chlorinated carbanilides at room temperature is a distinct advantage.

The quantity of surfactant employed is any quantity that brings about cleansing of any of the aforementioned surfaces, particularly skin. At least about 1 wt. %, desirably about 2, 3, 4, 5, 6, 7, or 8 wt. % of a surfactant or mixture of surfactants can be employed in the composition. Generally, at least about 20 wt. % of the surfactant present is anionic surfactant. The maximum amount of surfactant is not unduly significant and is generally a matter of other characteristics of the composition and cost. Generally, a composition having less than about 30, 25, 20, 15 or 12 wt. % of surfactant or mixture is desirable. Generally, compositions having any surfactants such as anionics for example, sodium laureth sulfate (SLES), alpha olefin sulfonate (AOS), betaines such as cocoamidopropyl betaine (CAPB) and nonionic such as alkyl polyglucoside (APG) therein are preferred. In calculating the weight of surfactant therein, neither the solubilizer nor cosolubilizer of this invention is counted in the surfactant total.

With respect to the carbanilides, particularly TCC, any antibacterial effective amount can be employed in the composition. Generally, at least about 0.01 wt. %, desirably above about 0.02, 0.03, 0.04 and 0.05 will bring about good results. Generally, no more than about 1.0, 0.9, 0.75 wt. % or 0.50 carbanilide is employed. The upper limit is at least somewhat dependent on the solubility of the carbanilide in the composition.

The solubilizing amide group of compound is present at quantities sufficient to bring about the above identified desirable properties. Generally, a minimum amount is about 0.25, 0.33, 0.4 or 0.5 wt. % of the composition. A maximum amount is determined by its effect on other characteristics of the formulation and cost but is generally no more than about 5, 4, or 3 wt. % of the composition.

The cosolubilizing long chain ethoxylated alcohol is present at quantities sufficient to assist in the achievement of the desirable formulation properties. Generally, a minimum amount is about 0.25, 0.33, 0.4 or 0.5 wt. % of the composition. A maximum amount is determined by its effect on the characteristics of the formulation and cost but is generally no more than about 5, 4 or 3 wt. % of the composition. When used together, the amide is preferably about 0.3 to 3 times the weight of the alcohol, more preferably about 0.7 to 1.3.

It has been found that the carbanilide, particularly a halogenated carbanilide can be readily processed into

the final fluid system by adding it to a fragrance when employed in the final fluid system. Fragrance(s) heavily organic in nature and comprising various odor causing components including oftentime one or more phenolic compounds make an excellent solubilizing system for the carbanilide (preferably halogenated), the amide solubilizing component, and, if employed, the cosolubilizing long chain ethoxy alcohol. Such mixture is made at room temperature and does not ordinarily require warming so as to significantly reduce the quantity of chloroanilines produced from chlorinated carbanilides under hydrolyzing conditions. Additionally, it is believed that the presence of the fragrance in combination with the amide assists in reducing the quantity of chloroanilines produced from hydrolyzing conditions of halogenated carbanilides, particularly TCC. In this composition of fragrance, carbanilide, amide and optionally alcohol, the approximate quantities are 5-60 wt. %, 1-20 wt. %, 1-85 wt. % and 0-75 wt. %, respectively.

Various other material can be present in the composition including emollients, colorants, thickeners, anti-inflammatories, chelators, and the like.

The fluid systems of this invention can be prepared in the following manner.

A. Sodium Laureth Sulfate (SLES) Based Shower Gel

1. Prepare TCC Premix (Promidium Co, Laureth-7, Perfume and TCC) in a separate premix tank. Follow the order of addition to obtain clear solution at room temperature. It is preferable to not heat the solution to avoid or minimize the formation of chloroanilines.
Order or Addition

- Promidium Co
- Laureth-7
- Perfume
- TCC

Mix thoroughly to obtain a clear solution.
If TCC as added to Promidium Co before the latter is blended with Laureth-7 and
Perfume, it will be difficult to get a clear solution at room temperature.

2. Add deionized water to the main tank

3. Add to the deionized water
EDTA
Dyes
Maintain agitation at low speed, Ensure at least five minutes dispersion time between each ingredient.

4. In a separate premix tank, dissolve a Polyquaternium, preferably Polyquat 7, if employed, with deionized water (ratio 50/50) and transfer the solution to the main tank.

5. Add Coco Amido Propyl Betaine to the main tank under slight agitation.

6. Add SLES under slight agitation. Avoid or minimize foam formation by controlling agitation speed.

7. Add alkyl polyglycoside heated to 40-50°C. under slight agitation.

8. Add TCC premix and increase the agitation speed till the whole solution is homogeneous.

9. Adjust pH to the specifications with an organic acid such as citric acid solution.

10. Add preservative under agitation to ensure thorough mixing.

11. Adjust viscosity to the specifications with viscosity modifying agent such as sodium chloride solution.

B. Alpha Olefin Sulfonate Based Shower Gel

1. Prepare TCC Premix (Promidium Co, Laureth-7, Perfume and TCC) in a separate premix tank. Follow the order to addition strictly, to obtain clear solution at room temperature. It is preferable to not heat the solution to avoid formation of chloroanilines.
<u>Order of Addition</u>

- Promidium Co
- Laureth-7
- Perfume
- TCC

Mix thoroughly to obtain a clear solution.
If TCC is added to Promidium Co before the latter is blended with Laureth-7 and Perfume, it will be very difficult to get a clear solution at room temperature.

2. Add deionized water to the main tank.

3. Add to the deionized water add EDTA, dyes, Glycerine Aloe Vera Gel and Silk Proteins.
Maintain agitation at low speed. Ensure five minutes dispersion time between each ingredient.

4. In a separate premix tank, dissolve Polyquatemium, preferably Polyquat 7, if employed with deionized water (ratio 50/50) and transfer the solution to the main tank.

5. Add AOS (Alpha Olefin Sulfonate) under slight agitation. Avoid foam formation by controlling agitation speed.

6. Add Coco Amido Propyl Betaine to the main tank under slight agitation.

7. Add TCC premix and increase the agitation speed till the whole solution is homogeneous.

8. Adjust pH to the specifications with citric acid solution.

9. Add preservative under agitation to ensure thorough mixing.

10. Adjust viscosity to the specifications with sodium chloride solution.

The following are examples of the invention. These are illustrative of the invention scope.

**<u>Example 1</u>**

| Ingredient Name | Weight % |
|---|---|
| | |
| Sodium Laureth Sulfate | 8.2 |
| Cocamidopropyl Betaine | 3.0 |
| Lauryl Polyglucose | 1.1 |
| Perfume | 1.0 |
| Sodium Chloride | 0.9 |
| Laureth Alcohol | 0.5 |

(continued)

| Ingredient Name | Weight % |
|---|---|
| Laureth-7 | 0.3 |
| PPG-2 Hydroxyethyl Cocamide | 0.3 |
| Sodium Sulfate | 0.3 |
| Polyquaternium-7 | 0.2 |
| Glycerin | 0.1 |
| Tetrasodium EDTA | 0.08 |
| Triclocarban | 0.075 |
| Citric Acid | 0.04 |
| Preservatives, etc. | 0.27 |
| Water | Q.S. |
| **TOTAL** | 100 |

**Example 2**

| Ingredient Name | Weight % |
| --- | --- |
| | |
| Sodium Laureth Sulfate | 8.2 |
| Cocamidopropyl Betaine | 3.2 |
| Lauryl Polyglucose | 1.1 |
| Perfume | 1.0 |
| Glycol Distearate | 0.9 |
| Laureth-7 | 0.7 |
| PPG-2 Hydroxyethyl Cocamide | 0.7 |
| Sodium Chloride | 0.6 |
| Laureth-4 | 0.5 |
| Laureth Alcohol | 0.4 |
| Sodium Sulfate | 0.3 |
| Polyquaternium-7 | 0.2 |
| Triclocarban | 0.15 |
| Tetrasodium EDTA }<br>Citric Acid }<br>Formic Acid }<br>Preservatives, etc. } | 0.4 |
| Water | Q.S. |
| **TOTAL** | 100 |

**Example 3**

| Ingredient Name | Weight % |
|---|---|
| | |
| Sodium $C_{14-16}$ Olefin Sulfonate | 8.1 |
| Cocamidopropyl Betaine | 2.4 |
| PPG-2 Hydroxyethyl Cocamide | 2.0 |
| Sodium Chloride | 1.8 |
| Laureth-7 | 1.0 |
| Perfume | 0.4 |
| Citric Acid | 0.3 |
| Sodium Sulfate | 0.3 |
| Triclocarban | 0.15 |
| Tetrasodium EDTA }<br>Polyquaternium-7 }<br>Preservatives, etc. } | 0.4 |
| Water | Q.S. |
| **TOTAL** | 100 |

**Example 4**

Using the formulation of Example 1 or Example 2 but with varying amounts of TCC, the amide wherein R is cocoyl, p is 2 and n is 1, and the alcohol wherein R' is lauryl and q is 7, antibacterial efficacy is measured using a cup scrub method on humans against the composition without TCC. The presence of Promidium Co and/or laureth-7 does not significantly affect antibacterial efficacy.

| Composition | Wt. % TCC | Wt. % Promidium Co | Wt. % Laureth-7 | Wt. % Fragrance | Antibacterial Efficacy, Log Reduction | Antibacterial Efficacy, Hours |
|---|---|---|---|---|---|---|
| | | | | | | |
| Example 1 | 0.075 | 0.34 | 0.34 | 1 | >1 | 8 |
| Example 2 | 0.15 | 0.67 | 0.67 | 1 | >1 | 24 |
| Example 1 | 0.225 | 1.01 | 1.01 | 1 | >1 | 24 |

As shown by this data, TCC brings about a greater than 1 log reduction of bacteria as measured against a composition without TCC.

## Claims

**1.** An aqueous fluid composition, which comprises:

a. cleansing effective amount of a surfactant or mixture thereof,
b. an antibacterial effective amount of a carbanilide compound or mixture thereof,
c. at least about 0.25 wt. % of composition of a compound or mixture thereof of the formula

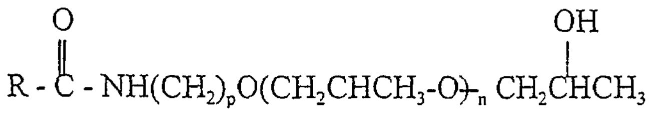

wherein R is alkyl or alkenyl of about 8 to about 20 carbon atoms, n is an integer of 1 to about 8, and p is 2 or 3.

**2.** The composition in accordance with claim 1 wherein the carbanilide is TCC.

**3.** The composition in accordance with claim 1 wherein in component c, p is 2 and n is 1.

**4.** The composition in accordance with claim 1 wherein additionally present is an alcohol compound or mixture of the formula

$$R'(CH_2CH_2O)_qH$$

wherein R' is alkyl or alkenyl of 10 to 15 carbon atoms and q is 7 to 9.

**5.** The composition in accordance with claim 3 wherein R is cocoyl.

**6.** The composition in accordance with claim 4 wherein R' is lauryl and q is 7.

**7.** The composition in accordance with claim 6 wherein

a. the carbanilide is TCC, and
b. in component c, R is about 12 to 16 carbon atoms, p is 2 and n is 1.

**8.** The composition in accordance with claim 7 wherein

a. surfactant or mixture thereof is at least about 5 wt. %,
b. TCC is at least about 0.03 wt. %
c. Component c of claim 1 wherein R is cocoyl is at least about 0.25 wt. %, and
d. the alcohol is at least about 0.25 wt. %.

**9.** A method of applying to a surface the composition of claim 1 after or concomitantly with dilution by water and obtaining a combination of surface cleansing and antibacterial efficacy.

**10.** The method in accordance with claim 9 wherein the surface is skin.

**11.** A method of applying to a surface the composition of claim 8 after or concomitantly with dilution by water and obtaining a combination of surface cleansing and antibacterial efficacy.

**12.** The method in accordance with claim 11 wherein the surface is skin.

**13.** A composition comprising

a. a fragrance
b. an antibacterial carbanilide
c. a compound or mixture thereof of the formula

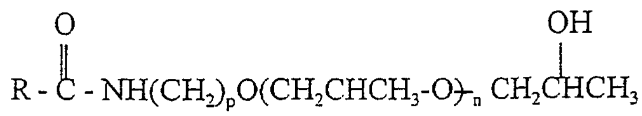

wherein r is alkyl or alkenyl of about 8 to about 20 carbon atoms, n is an integer of 1 to about 8, and p is 2 or 3.

**14.** The composition in accordance with claim 13 wherein the carbanilide is TCC.

**15.** The composition in accordance with claim 13 wherein n is 1 and p is 2.

**16.** The composition in accordance with claim 13 wherein additionally present is an alcohol compound or mixture thereof of the formula

$$R'(CH_2CH_2O)_qH$$

wherein R' is alkyl or alkenyl of 10 to 15 carbon atoms, and q is 7, 8 or 9.

**17.** The composition in accordance with claim 16 wherein

a. the carbanilide is TCC
b. R is about 10 to 14 carbon atoms, n is 1 and p is 2
c. R' is lauryl and q is 7.

**Patentansprüche**

**1.** Wässrige Fluidzusammensetzung, die

a. eine reinigend wirkende Menge Tensid oder einer Mischung desselben,

b. eine antibakteriell wirkende Menge Carbanilidverbindung oder einer Mischung derselben,

c. mindestens etwa 0,25 Gew.-% der Zusammensetzung an einer Verbindung mit der Formel

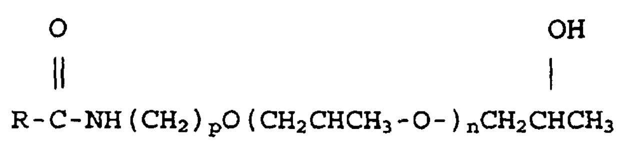

oder einer Mischung derselben umfasst, in der R Alkyl oder Alkenyl mit etwa 8 bis etwa 20 Kohlenstoffatomen ist, n eine Zahl von 1 bis etwa 8 ist und p 2 oder 3 ist.

**2.** Zusammensetzung nach Anspruch 1, bei der das Carbanilid TCC ist.

**3.** Zusammensetzung nach Anspruch 1, bei der in Komponente c p 2 ist und n 1 ist.

**4.** Zusammensetzung nach Anspruch 1, bei der zusätzlich eine Alkoholverbindung mit der Formel

$$R'(CH_2CH_2O)_qH$$

oder eine Mischung vorhanden ist, in der R' Alkyl oder Alkenyl mit 10 bis 15 Kohlenstoffatomen ist und q 7 bis 9 ist.

**5.** Zusammensetzung nach Anspruch 3, bei der R Cocoyl ist.

**6.** Zusammensetzung nach Anspruch 4, bei der R' Lauryl ist und q 7 ist.

**7.** Zusammensetzung nach Anspruch 6, bei der

a. das Carbanilid TCC ist und

b. in Komponente c R etwa 12 bis 16 Kohlenstoffatome aufweist, p 2 ist und n 1 ist.

**8.** Zusammensetzung nach Anspruch 7, bei der

a. Tensid oder eine Mischung desselben mindestens 5 Gew.-% ausmacht,

b. TCC mindestens etwa 0,03 Gew.-% ausmacht,

c. Komponente c gemäß Anspruch 1, wobei R Cocoyl ist, mindestens etwa 0,25 Gew.-% ausmacht und

d. der Alkohol mindestens etwa 0,25 Gew.-% ausmacht.

**9.** Verfahren zum Aufbringen der Zusammensetzung gemäß Anspruch 1 auf eine Oberfläche nach oder gleichzeitig mit der Verdünnung durch Wasser und Erhalten einer Kombination von oberflächenreinigender und antibakterieller Wirksamkeit.

**10.** Verfahren nach Anspruch 9, bei der die Oberfläche Haut ist.

**11.** Verfahren zum Aufbringen der Zusammensetzung gemäß Anspruch 8 auf eine Oberfläche nach oder gleichzeitig mit der Verdünnung durch Wasser und Erhalten einer Kombination von oberflächenreinigender und antibakterieller Wirksamkeit.

**12.** Verfahren nach Anspruch 11, bei der die Oberfläche Haut ist.

**13.** Zusammensetzung, die

a. Duftstoff,

b. antibakterielles Carbanilid,

c. eine Verbindung der Formel

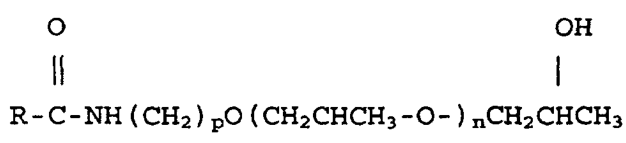

oder eine Mischung derselben umfasst, in der R Alkyl oder Alkenyl mit etwa 8 bis etwa 20 Kohlenstoffatomen ist, n eine Zahl von 1 bis etwa 8 ist und p 2 oder 3 ist.

**14.** Zusammensetzung nach Anspruch 13, bei der das Carbanilid TCC ist.

**15.** Zusammensetzung nach Anspruch 13, bei der n 1 ist und p 2 ist.

**16.** Zusammensetzung nach Anspruch 13, bei der zusätzlich eine Alkoholverbindung mit der Formel

$$R' (CH_2CH_2O)_qH$$

oder eine Mischung derselben vorhanden ist, in der R' Alkyl oder Alkenyl mit 10 bis 15 Kohlenstoffatomen ist und q 7, 8 oder 9 ist.

**17.** Zusammensetzung nach Anspruch 16, bei der

a. das Carbanilid TCC ist,

b. R etwa 10 bis 14 Kohlenstoffatome aufweist, n 1 ist und p 2 ist,

c. R' Lauryl ist und q 7 ist.

**Revendications**

**1.** Composition fluide aqueuse, qui comprend :

a. une quantité efficace de nettoyant d'un agent tension actif ou d'un mélange de celui-ci,
b. une quantité efficace d'antibactérien d'un composé de carbanilide ou d'un mélange de celui-ci,
c. au moins environ 0,25 % en poids de la composition d'un composé ou d'un mélange de celui-ci de formule

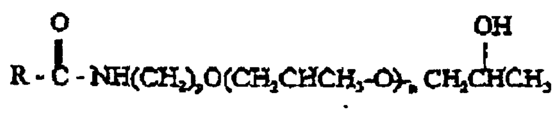

dans laquelle R est un alkyle ou un alkényle d'environ 8 à environ 20 atomes de carbone, n est un nombre entier de 1 à environ 8, et p est égal à 2 ou 3.

**2.** Composition selon la revendication 1, dans laquelle le carbanilide est du TCC.

**3.** Composition selon la revendication 1, dans laquelle dans le composant c, p est égal à 2 et n est égal à 1.

**4.** Composition selon la revendication 1, dans laquelle est présent en outre un composé d'alcool ou un mélange de formule

$$R'(CH_2CH_2O)_qH$$

dans laquelle R' est un alkyle ou un alkényle de 10 à 15 atomes de carbone et q est compris entre 7 et 9.

**5.** Composition selon la revendication 3, dans laquelle R est du cocoyl.

**6.** Composition selon la revendication 4, dans laquelle $R^1$ est du lauryl et q est égal à 7.

**7.** Composition selon la revendication 6, dans laquelle :

a. le carbanilide est du TCC, et
b. dans le composant c, R est constitué d'environ 12 à 16 atomes de carbore, p est égal à 2 et n est égal à 1.

**8.** Composition selon la revendication 7, dans laquelle

a. le tensioactif ou le mélange de celui-ci représente au moins environ 5 % en poids,
b. le TCC représente au moins environ 0,03 % en poids,

c. le composant c selon la revendication 1 dans lequel R est du cocoyl représente au moins environ 0,25 % en poids, et
d. l'alcool représente au moins environ 0,25 % en poids.

**9.** Procédé d'application sur une surface de la composition selon la revendication 1 après ou simultanément avec dilution par de l'eau et d'obtention dune combinaison de nettoyant de surface et d'antibactériens efficace.

**10.** Procédé selon la revendication 9, dans lequel la surface est la peau.

**11.** Procédé d'application sur une surface de la composition selon la revendication 8 après ou simultanément avec dilution par de l'eau et d'obtention d'une combinaison de nettoyant de surface et d'antibactériens efficace.

**12.** Procédé selon la revendication 11, dans lequel la surface est la peau.

**13.** Composition comprenant :

a. une fragrance,
b. un carbanilide antibactérien,
c. un composé ou un mélange de celui-ci de formule

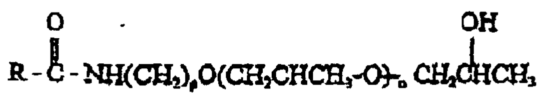

dans laquelle R est un alkyle ou un alkényle d'environ 8 à environ 20 atomes de carbone, n est un nombre entier de 1 à environ 8, et p est égal à 2 ou 3.

**14.** Composition selon la revendication 13, dans laquelle le carbanilide est du TCC.

**15.** Composition selon la revendication 13, dans laquelle n est égal à 1 et p est égal à 2.

**16.** Composition selon la revendication 13, dans laquelle en outre est présent un composé d'alcool ou un mélange de celui-ci de formule

$$R'(CH_2CH_2O)_qH$$

dans laquelle R' est un alkyle ou un alkényle de 10 à 15 atomes de carbone, et q est égal à 7, 8 ou 9.

**17.** Composition selon la revendication 16, dans laquelle

a. le carbanilide est du TCC
b. R contient environ 10 à 14 atomes de carbone, n est égal à 1 et p est égal à 2
c. R' est du lauryl et q est égal à 7.